# EUROPEAN PATENT SPECIFICATION

(11) **EP 1 703 901 B1**
(45) Date of publication and mention of the grant of the patent: **08.10.2008**
(21) Application number: 05701832.7
(22) Date of filing: 11.01.2005
(51) Int. Cl.: A61K 31/055, A61K 31/05, A61P 31/14

(54) **PHARMACEUTICAL COMPOSITION COMPRISING 2,4-DICHLOROBENZYL ALCOHOL AND AMYLMETACRESOL**
PHARMAZEUTISCHE ZUSAMMENSETZUNG ENTHALTEND 2,4-DICHLORBENZYLALKOHOL UND AMYLMETAKRESOL
COMPRENANT DE L'ALCOOL 2,4-DICHLOROBENZYLIQUE ET DE L'AMYLMETACRESOL

(30) Priority: 14.01.2004 GB 0400774
(43) Date of publication of application: 27.09.2006
(73) Proprietor: Reckitt Benckiser Healthcare (UK) Limited, Slough, Berkshire SL1 3UH (GB)
(72) Inventor: GIBB, Ian, Nottingham NG2 3AA (GB)
(74) Representative: O'Brien, Niall James
(86) International application number: PCT/GB2005/000062
(87) International publication number: WO 2005/067906

(56) References cited:
- WO-A-96/32934
- GB-A- 865 672
- US-A- 3 123 528
- FROSCH, FRANZ ET AL: "Investigation of the sensitivity of microorganism to active agents in sore throat lozenges" DEUTSCHE APOTHEKER ZEITUNG , 118(21), 758-60 CODEN: DAZEA2; ISSN: 0011-9857, 1978, XP008055892
- EDITIONS DU VIDAL ED-EDITIONS DU VIDAL ED - EDITIONS DU VIDAL: "VIDAL 1997, PASSAGE" 1997, DICTIONNAIRE VIDAL 1997, PARIS, EDITIONS DU VIDAL, FR, PAGE 1565 , XP002355045 ISBN: 2-85091-075-9 page 1565

## Description

The present invention relates to the use of formulations of 2,4-dichlorobenzyl alcohol (or 2,4-DCBA) and amylmetacresol (or AMC) in the treatment or prevention of Severe Acute Respiratory Syndrome (SARS) viral infections.

Severe Acute Respiratory Syndrome (SARS) is a viral respiratory illness caused by a novel coronavirus, termed SARS-associated coronavirus (SARS-CoV). SARS was first reported in China in 2003 and subsequently the illness spread to more than twenty two countries in North America, South America, Europe and Asia. During the SARS outbreak of 2003, over 8,000 people worldwide became sick with SARS; of these, nearly 800 died. In particular, the mortality rate exceeded 60% in the over 60 age group. Although the SARS outbreak of 2003 was contained, it is possible that the disease may re-emerge. In fact, in January 2004, a further case of SARS has been reported in China.

Typically, SARS begins with a high fever with a body temperature of greater than 38 °C. Other symptoms may include a dry cough, body aches, shortness of breath and an overall feeling of discomfort. Generally, most patients also develop pneumonia.

The main way SARS seems to spread is by close person to person contact. It is believed the virus is most readily transmitted by respiratory droplets which may be produced when an infected person coughs or sneezes. Such respiratory droplets may be transported through the air and deposited on the mucous membranes of the mouth, nose or eyes of persons who are nearby. The virus may also be spread when a person touches a surface contaminated with infectious droplets and then touches his or her mouth, nose or eyes.

Suitably, the spread of SARS is typically contained by isolating those people who have been infected with the virus and quarantining those people who have been exposed to the virus and may be infected but not ill. Consequently, there exists a need for the prophylactic and curative treatment of SARS-associated coronavirus (SAR-CoV) viral infections.

The compound 2,4-dichlorobenzyl alcohol is known as an antiseptic agent, i.e. as an antibacterial and antifungal agent, see for example Martindale "The Extra Pharmacopoeia" 28th edition, page 561, The Pharmaceutical Press (1982). Amylmetacresol or 6-pentyl-m-cresol is also known as a disinfectant used in mouthwashes or lozenges in combination with 2,4-dichlorobenzyl alcohol to treat mouth infections, see for example Martindale "The Extra Pharmacopoeia" 28th edition, page 549, The Pharmaceutical Press (1982). An antiseptic is defined in The Concise Oxford Dictionary (Oxford University Press, Oxford (1982)) as an agent which counters the development of sepsis, especially by preventing the growth of bacteria. Sepsis is defined in Black's Medical Dictionary (A & C Black, London (1990)) as poisoning by the products of the growth of micro-organisms in the body, and the general symptoms which accompany it are those of inflammation. Neither 2,4-dichlorobenzyl alcohol or amylmetacresol has previously been shown to demonstrate activity against SARS-associated coronavirus.

Unexpectedly, it has now been discovered that a formulation of 2,4-dichlorobenzyl alcohol and amylmetacresol exhibits virucidal activity against SARS-associated coronavirus, in particular the phenotype Urbani SARS-associated coronavirus.

According to a first aspect, the present invention provides the use of 2,4-dichlorobenzyl alcohol in combination with amylmetacresol in the manufacture of a medicament (ie a pharmaceutical composition) for the treatment or prevention of viral infections caused by SARS-associated coronavirus (SAR-CoV), in particular Urbani SARS-associated coronavirus. Preferably, the pharmaceutical composition is effective for the treatment of viral infections caused by SARS-associated coronavirus, in particular Urbani SARS-associated coronavirus.

Suitably, the pharmaceutical composition may be adapted for administration to the mucosa of a patient by any appropriate route, for example by the nasal, ocular, vaginal, rectal or oral routes. Such compositions may be prepared by any method known in the art of pharmacy, for example by admixing the active ingredients with the carrier(s) or excipient(s) under sterile conditions. Preferably, the pharmaceutical composition is adapted for nasal, ocular or oral administration, especially oral administration.

Suitably, the pharmaceutical composition is in the form of a liquid (i.e. solution, suspension, an emulsion, syrup, gel) or a masticable or suckable solid dosage form. For the preparation of solutions, syrups and gels suitable excipients include water, polyols and sugars especially liquid glucose and/or liquid sucrose. For the preparation of suspensions, suitable excipients include suspension oils (e.g. vegetable oils). Suitable emulsions include oil-in-water or water-in-oil emulsions. For the preparation of suckable solid dosage forms, for example lozenges, the 2,4-dichlorobenzyl alcohol and amylmetacresol may be blended into a molten lozenge base (e.g. a mixture of sugar and liquid glucose plus excipients or a non-sugar base such as isomalt plus optional excipients) and the mixture drawn into a cylindrical mass from which the lozenges are formed. Alternatively, the masticable or suckable solid dosage form may be in the form of a masticable or suckable tablet, capsule, pastille or gum, for example chewing gum, which may be prepared by techniques well known to those skilled in the art.

Highly preferred pharmaceutical compositions are in the form of a suckable or masticable solid dosage form, such as a lozenge or a masticable or suckable tablet, a fast melting tablet (Meltlet^{™}) capsule or pastille as described herein. Suitably, such dosage forms are adapted to release a therapeutically effective amount of 2,4-dichlorobenzyl alcohol and amylmetacresol to the surface of the oral cavity of a patient in need thereof. An especially preferred solid dosage form is in the form of a lozenge which may be prepared by cooling a heated lozenge sugar base (e.g. sucrose and liquid glucose) or a heated lozenge sugar alcohol base (i.e. lycasin, isomalt and/or sorbitol), 2,4-dichlorobenzyl alcohol, amylmetacresol and other excipients to form solid lozenges.

Preferably, the composition is in the form of an oral dosage form such that when the dosage form is administered to the oral cavity of a patient the dosage form releases a therapeutically effective amount of 2,4-dichlorobenzyl alcohol and amylmetacresol to the surface of the oral cavity, particularly the mucous membrane of the throat. Preferably, the pharmaceutical composition is a sugar based (e.g. sucrose and/or glucose), especially sucrose based, or sugar alcohol based (e.g. isomalt, lycasin or sorbitol) solution, suspension, emulsion, syrup, gel or suckable or masticable solid dosage form.

When the pharmaceutical composition is in the form of a liquid (i.e. solution, suspension, emulsion, syrup, or gel), the composition may be administered directly in liquid form to the oral cavity. Alternatively, such a solution, suspension, emulsion, syrup or gel may be encapsulated with a material (i.e. a hydroxy propyl methyl cellulose film or gellatin film) adapted to disintegrate in the mouth so as to form a discrete dosage unit. Conveniently, following administration of such a dosage form the active components of the solution, suspension, emulsion, syrup or gel are released in the oral cavity following dissolution of or rupturing of the encapsulating material.

Suitably, when the pharmaceutical composition is in the form of a liquid, the pharmaceutical composition is preferably in the form of a solution, syrup or suspension. Suitably, when the pharmaceutical composition is in the form of a solution the composition may be packed into a dispensing container fitted with a spray mechanism which enables the composition to be sprayed into the ora! cavity, particularly onto the mucous membrane of the throat, as a fine spray.

Suitably, where the pharmaceutical composition is in the form of a liquid, for example solution, suspension, gel or syrup, preferably the 2,4-dichlorobenzyl alcohol is present in an amount of greater than or equal to 10 µg/ml, more preferably greater than or equal to 0.1 mg/ml, most preferably greater than or equal to 1 mg/ml based on the total volume of the pharmaceutical composition.

Suitably, where the pharmaceutical composition is in the form of a liquid, for example solution, suspension, gel or syrup, preferably the 2,4-dichlorobenzyl alcohol is present in an amount of less than or equal to 10 mg/ml, more preferably less than or equal to 7 mg/ml, most preferably less than or equal to 5 mg/ml based on the total volume of the pharmaceutical composition.

Suitably, when the pharmaceutical composition is in the form of a liquid, preferably the amylmetacresol is present in an amount of greater than or equal to 10 µg/ml, preferably greater than or equal to 50 µg/ml, most preferably greater than or equal to 100 µg/ml, preferably greater than or equal to 400 µg/ml based on the total volume of the pharmaceutical composition.

Suitably, when the pharmaceutical composition is in the form of a liquid, preferably the amylmetacresol is present in an amount of less than or equal to 5 mg/ml, preferably less than or equal to 2.0 mg/ml, most preferably less than or equal to 1 mg/ml based on the total volume of the pharmaceutical composition.

Suitably, when the pharmaceutical composition is in the form of a liquid then the total volume of the pharmaceutical composition is greater than or equal to 2 ml, preferably greater than or equal to 3 ml, most preferably greater than or equal to 5 ml. Suitably, when the pharmaceutical composition is in the form of a liquid then the total volume of the pharmaceutical composition is less than or equal to 10 ml, preferably less than or equal to 8 ml, most preferably less than or equal to 7 ml.

Suitably, when the pharmaceutical composition is in the form of a solution or a suspension, the main component of the liquid phase comprises water. Preferably, such solutions and suspensions comprise greater than or equal to 2 ml, preferably greater than or equal to 3 ml, most preferably greater than or equal to 5 ml of water. Preferably, such solutions and suspensions comprise less than or equal to 10 ml, preferably less than or equal to 8 ml, most preferably less than or equal to 7 ml of water.

Suitably, when the pharmaceutical composition is in the form of a syrup, the main component of the liquid phase comprises a liquid sugar. By the term "liquid sugar" as used herein, we mean a sugar dissolved in an appropriate solvent, preferably the solvent comprises water. Most preferred liquid sugars include liquid glucose, comprising an aqueous solution of glucose (e.g. 65 to 90% by wt of glucose), and liquid sucrose, comprising an aqueous solution of sucrose (e.g. 55 to 80% by wt sucrose). Suitably, the syrup comprises greater than or equal to 2 ml, preferably greater than or equal to 3 ml, most preferably greater than or equal to 5 ml of a liquid sugar. Preferably, the syrup comprises less than or equal to 10 ml, preferably less than or equal to 8 ml, most preferably less than or equal to 7 ml of a liquid sugar.

Suitably, when the pharmaceutical composition is in the form of a liquid, then the liquid phase (i.e. water for suspensions and solutions, and liquid sugar for syrups) is present in an amount of greater than or equal to 90% by wt, more preferably greater than or equal to 95% by wt based on the total weight of the pharmaceutical composition.

Preferably, the pharmaceutical composition is in the form of a suckable or masticable solid dosage form, particularly a lozenge or a masticable or suckable tablet, capsule or pastille. The term "lozenge" as used herein means all dosage forms where the product is formed by cooling a sugar-based (e.g. sucrose and/or glucose) or sugar alcohol based (e.g. sorbitol and/or isomalt and/or lycasin) molten mass containing the active ingredient. A preferred suckable solid dosage form is a lozenge prepared by cooling a heated lozenge base comprising sugar, especially sucrose and/or glucose, 2,4-dichlorobenzyl alcohol and amylmetacresol and optionally other excipients to form solid lozenges, for example acidity regulators, opacifiers, stabilising agents, buffering agents, flavourings, sweeteners, colouring agents and preservatives. Lozenges may be formed by standard techniques known to those skilled in the art as disclosed in European Patent no. 0862424B (PCT/EP96/05208) by The Boots Company PLC.

For example, lozenges may be formed by heating the lozenge base (e.g. a mixture of sucrose and liquid glucose or an aqueous mixture of lycasin and/or isomalt and/or sorbitol) under vacuum to remove excess water and the remaining components (e.g. 2,4-dichlorobenzyl alcohol, amylmetacresol and other optional excipients) are then blended into the mixture. The resulting mixture is then drawn into a continuous cylindrical mass from which the individual lozenges are formed. The lozenges are then cooled, subjected to a visual check and packed into suitable packaging (i.e. a blister pack). The patient removes the lozenge by applying pressure to the blister to force the lozenge to rupture and pass through the blister seal. Lozenges will normally be sucked by the patient to release the 2,4-dichlorobenzyl alcohol and the amylmetacresol.

Masticable solid dosage forms may be prepared by the methods used to prepare chewable candy products or chewing gums. For example, a chewable solid dosage form may be prepared from an extruded mixture of a sugar base (e.g. sucrose and liquid glucose) or a sugar alcohol base (e.g. lycasin, isomalt or sorbitol) to which 2,4-dichlorobenzyl alcohol and amylmetacresol has been added with optional addition of whipping agents, humectants, lubricants, flavourings and colourings (see Pharmaceutical Dosage Forms: Tablets, Volume 1, Second Edition edited by H A Lieberman, L Lachman and J B Schwartz published in 1989).

Masticable or suckable tablets may be made from compressed powders or granules or pastes by techniques well known to those skilled in the art.

Suitably, where the pharmaceutical composition is in the form of a suckable or masticable solid dosage form (i.e. lozenge), preferably the 2,4-dichlorobenzyl alcohol is present in an amount of greater than or equal to 0.2 mg, more preferably greater than or equal to 0.5 mg, even more preferably greater than or equal to 1.0 mg, most preferably greater than or equal to 1.5 mg based on the total weight of the pharmaceutical composition.

Suitably, where the pharmaceutical composition is in the form of a suckable or masticable solid dosage form (i.e. lozenge), preferably the 2,4-dichlorobenzyl alcohol is present in an amount of less than or equal to 5 mg, more preferably less than or equal to 3 mg, even more preferably less than or equal to 2 mg, most preferably less than or equal to 1.75 mg based on the total weight of the pharmaceutical composition.

Suitably, where the pharmaceutical composition is in the form of a suckable or masticable solid dosage form (i.e. lozenge), preferably the amylmetacresol is present in an amount of greater than or equal to 50 µg, more preferably greater than or equal to 100 µg, even more preferably greater than or equal to 250 µg, most preferably greater than or equal to 500 µg based on the total weight of the pharmaceutical composition.

Suitably, where the pharmaceutical composition is in the form of a suckable or masticable solid dosage form (i.e. lozenge), preferably the amylmetacresol is present in an amount of less than or equal to 1,500 µg, more preferably less than or equal to 1,000 µg, even more preferably less than or equal to 800 µg, most preferably less than or equal to 700 µg based on the total weight of the pharmaceutical composition.

Suitably, where the pharmaceutical composition is in the form of a suckable or masticable solid dosage form (i.e. lozenge), preferably the total weight of the pharmaceutical composition is greater than or equal to 1 g, more preferably greater than or equal to 1.5 g, most preferably greater than or equal to 2 g.

Suitably, where the pharmaceutical composition is in the form of a suckable or masticable solid dosage form (i.e. lozenge), preferably the total weight of the pharmaceutical composition is less than or equal to 4 g, more preferably less than or equal to 3.5 g, most preferably less than or equal to 3 g.

It will be appreciated by those skilled in the art that the remainder of the suckable or masticable solid dosage form apart from 2,4-dichlorobenzyl alcohol and amylmetacresol consists essentially of (i.e. greater than or equal to 90% by weight, more preferably greater than or equal to 95% by weight, even more preferably greater than or equal to 97% by weight, most preferably greater than or equal to 98% by weight, especially about 99% by weight of a sugar base), of a sugar base, particularly sucrose and/or glucose, or a sugar alcohol base, particularly sorbitol and/or isomalt and/or lycasin, as defined herein. The sugar base or sugar alcohol base is preferably acidulated as described herein. Suitably, when the pharmaceutical composition includes an acidic sugar base or an acidic sugar alcohol base, then such acidic bases in total are preferably present in the same preferred amounts as the sugar base and sugar alcohol base, respectively.

In a particularly preferred embodiment of the present invention there is provided a Strepsils^{™} like formulation of 2,4-dichlorobenzyl alcohol and amylmetacresol for the treatment or prevention of SARS-associated coronavirus viral infections.

Preferably, the amylmetacresol is present in an amount of greater than or equal to 0.01% by weight, more preferably greater than or equal to 0.02% by weight, most preferably greater than or equal to 0.025% by weight based on the total weight of the pharmaceutical composition.

Preferably, the amylmetacresol is present in an amount of less than or equal to 0.5% by weight, more preferably less than or equal to 0.1% by weight, most preferably less than or equal to 0.05% by weight based on the total weight of the pharmaceutical composition.

Preferably, the 2,4-dichlorobenzyl alcohol is present in an amount of greater than or equal to 0.02% by weight, more preferably greater than or equal to 0.04% by weight, most preferably greater than or equal to 0.05% by weight based on the total weight of the pharmaceutical composition.

Preferably, the 2,4-dichlorobenzyl alcohol is present in an amount of less than or equal to 1% by weight, more preferably less than or equal to 0.2% by weight, most preferably less than or equal to 0.1% by weight based on the total weight of the pharmaceutical composition.

Preferably, the ratio by weight of amylmetacresol to 2,4-dichlorobenzyl alcohol in the pharmaceutical composition is in the range of 1:20, more preferably 1:10, even more preferably 1:5, most preferably 1:2.

It will be appreciated that the pharmaceutical composition in any form (i.e. liquid or solid) may include the above preferred % by weight amounts of 2,4-dichlorobenzyl alcohol and amylmetacresol and/or the preferred ratio by weight of 2,4-dichlorobenzyl alcohol to amylmetacresol.

Unexpectedly, it has additionally been found that the pharmaceutical composition of the present invention exhibits improved virucidal activity against SARS-associated coronavirus when present in an acidic environment. Suitably, according to a preferred aspect of the present invention, the pharmaceutical composition is in acidic form (i.e. acidulated). More preferably, the pharmaceutical composition comprises a suckable or masticable solid dosage form (i.e. a lozenge) in acidic form, especially a lozenge which includes an acidic sugar base (i.e. sucrose and/or glucose) or an acidic sugar alcohol base (i.e. sorbitol and/or isomalt and/or lycasin), more especially an acidic sugar base, most especially an acidic sucrose and/or glucose base.

Preferably, the pharmaceutical composition has a pH of less than or equal to 6.8, more preferably less than or equal to 6.5, most preferably less than or equal to 6.0. Preferably the pharmaceutical composition of the present invention has a pH of greater than or equal to 5.0, more preferably greater than or equal to 5.5. Suitably, when the pharmaceutical composition includes a separate acid preferably the combination has a pH of less than or equal to 5.0, more preferably less than or equal to 4.5, most preferably less than or equal to 4.0. Suitably, when the pharmaceutical composition includes a separate acid preferably the composition has a pH of greater than or equal to 2.0, more preferably greater than or equal to 2.5. It will be appreciated that when the pharmaceutical composition is in the form of a solid dosage form, then the pH of the pharmaceutical composition may be measured by dissolving and/or dispersing the composition in distilled water, preferably 5 ml of distilled water. The resultant aqueous dispersion and/or solution of the pharmaceutical composition preferably has a pH as defined in the preceding sentences. Most preferably, the pharmaceutical composition includes an acidic base (i.e. an acidic sugar base or an acidic sugar alcohol base), particularly an acidic sugar base, especially an acidic sucrose base.

Thus according to a preferred aspect, the pharmaceutical composition comprises 2,4-dichlorobenzyl alcohol and amylmetacresol in an acidic sugar (especially sucrose and/or glucose) or acidic sugar alcohol base (especially lycasin, isomalt and/or sorbitol), particularly an acidic sugar base, especially an acidic sucrose and glucose base, for the treatment or prevention of viral infections caused by SARS-associated coronavirus.

Preferably, when the composition includes an acidic sugar base or an acidic sugar alcohol base, the acidic base is the predominant component of the pharmaceutical composition. Preferably, the sugar or sugar alcohol is present in an amount of greater than or equal to 90% by weight, more preferably greater than or equal to 95% by weight, most preferably greater than or equal to 98% by weight based on the total weight of the pharmaceutical composition.

The acidic base may be formed by the inclusion of a separate acid to the pharmaceutical composition of the present invention. Suitable acids are organic acids, particularly citric, malic, maleic, fumaric, succinic, adipic and/or tartaric acid. Preferably, the acid is present in an amount of greater than or equal to 0.1% by wt, more preferably greater than or equal to 0.2% by wt, most preferably greater than or equal to 0.5% by wt based on the total weight of the pharmaceutical composition. Preferably, the acid is present in an amount of less than or equal to 2% by wt, more preferably less than or equal to 1.6% by wt, most preferably less than or equal to 1.4% by wt based on the total weight of the pharmaceutical composition.

It is also contemplated that the pharmaceutical compositions may include antioxidants, buffers, bacteriostats, solutes, suspending agents and thickening agents. Excipients which may be used include water, alcohols, polyols, glycerine and vegetable oils, for example.

The pharmaceutical compositions may contain preserving agents, solubilising agents, stabilising agents, wetting agents, emulsifiers, sweeteners, colorants, odorants, salts (substances of the present invention may themselves be provided in the form of a pharmaceutically acceptable salt), buffers, coating agents or antioxidants. They may also contain therapeutically active agents in addition to the substance of the present invention.

Dosages of the substance of the present invention can vary between wide limits, depending upon the age and condition of the individual to be treated, etc. and a physician will ultimately determine appropriate dosages to be used.

A typical pharmaceutical composition in the form of a suckable or masticable solid dosage form consists essentially of:

| | Ingredient | wt% |
|---|---|---|
| (a) | Acid | 0.2 to 1.6 |
| (b) | 2,4-dichlorobenzyl alcohol | 0.02 to 0.10 |
| (c) | Amylmetacresol | 0.01 to 0.05 |
| (d) | Excipients (flavouring, colouring, | 0.1 to 1.5 |
| | preservatives, etc.) | |
| (e) | Sugar or sugar alcohol base | Balance of composition, typically greater than or equal to 97% by weight |
| (f) | Water | Up to 4% by weight, preferably 1 to 3% by wt |

where the sum of components (a) to (f) represent greater than or equal to 98% by wt, more preferably greater than or equal to 99% by wt, most preferably substantially 100% by wt of the composition.

A highly preferred pharmaceutical composition in the form of a suckable or masticable solid dosage form comprises:
0.01 to 0.05% by weight amylmetacresol;
0.02 to 0.10% by weight 2,4-dichlorobenzyl alcohol; and
0 to 1.4% by weight organic acid, preferably 0.8% to 1.4% by weight organic acid;
greater than 95% by weight sugar, especially sucrose and/or glucose.

An alternative highly preferred composition in the form of a suckable or masticable solid dosage form comprises:
0.01 to 0.05°C by weight amylmetacresol
0.02 to 0.10% by weight 2,4-dichlorobenzyl alcohol; and
0 to 1.4% by weight organic acid, preferably 0.8% to 1.4% by weight organic acid;
greater than 95% by weight sugar alcohol, particularly sorbitol and/or isomalt and/or lycasin.

Suitably, as such a formulation has been used for two decades or more as Strepsils^{™} mouth lozenges to combat bacterial infections it is speculated that the mixture has little deleterious effect on mucosal cells of the patient. Conveniently, the mixture may however be used for the treatment or prevention of viral infections caused by SARS-associated coronavirus.

The invention will now be described by way of the following examples:

### Materials and Methods

### (a) Positive Control Sample

The following positive control sample including a surfactant (Triton X100) which is known to disrupt viral envelopes was prepared.

| | |
|---|---|
| Positive Control 1 (PC1): | Artificial saliva (0.1% NaHCO₃, 18% KH₂PO₄ and 0.1% gastric mucin in double distilled water, pH adjusted to 6.0 to 6.5) plus 0.1 % Triton X100 |

### (b) Negative Control Sample

The following negative control sample which did not include an active agent which disrupts viral envelopes were prepared.

| | |
|---|---|
| Negative Control 1 (NC1): | Artificial saliva (0.1% NaHCO₃, 18% KH₂PO₄ and 0.1% gastric mucin in double distilled water, pH adjusted to 6.0 to 6.5). |

Urbani SARS virus diluted in Dulbecco's Minimal Essential Medium (DMEM) at a final dilution of 10⁻².

### (c) Test Composition (TC)

A commercial tablet of Strepsils Original Throat Lozenge available from The Boots Company PLC which contained 1.2 mg 2,4-dichlorobenzyl alcohol and 0.6 mg amylmetacresol in a sucrose base (acidic) was dissolved in 5 ml of artificial saliva (0.1 % NaHCO₃, 18% KH₂PO₄ and 0.1 % gastric mucin in double distilled water) so that the mixture was pH adjusted to 6.0 to 6.5.

### Cell Cultures

C1008 cells (a clone of Vero 76) were used for virucidal tests and cells were cultivated in RPMI 1640 medium (available from Sigma Chemical Company, UK) supplemented with 10% heat-inactivated (56°C, 30 minutes) foetal calf serum (FCS), 2 mM L-glutamine, 100 µg/ml penicillin and 100 µg/ml streptomycin.

### Example 1 In-vitro activity

40 µl Urbani SARS-associated coronavirus (initial titre of 10⁵TCID₅₀/ml) was added to 360 µl of the test composition (TC), the negative control sample (NC1) and the positive control sample (PC1), respectively, as listed above. All solutions were thoroughly mixed and incubated at 37 °C and 22.9 °C for 30 seconds, 1 minute, 2 minutes and 8 minutes. The final titre of Urbani SARS-associated coronavirus after mixing was 10^{3.5} TCID₅₀/ml for the assay conducted at 22.9 °C and 10³ TCID₅₀/ml for the assay conducted at 37°C. The reaction between the virus and test composition or control samples was terminated by a dilution effect by adding 3.6 mls of media (DMEM). At these particular time periods, the respective mixture of virus/compound (110 µl) was titrated on the C1088 cell culture at ten fold dilutions across a 96 well plate and the plates incubated for 5 to 6 days at 37°C. The results were analysed for virus titre determined using the Karber method and are displayed in Tables 1 and 2 below.

**Table 1**

| The following table shows the log reduction in viral titre for both the test composition (PC1) and control compositions in the virucidal assay conducted at 37 °C. | | | | | |
|---|---|---|---|---|---|
| | Contact Time | | | | |
| Test/Control | Time (minutes) | | | | |
| Compounds | 0.5 | 1 | 2 | 4 | 8 |
| Strepsils Original Throat Lozenges (TC) | 1.0 | 1.5 | 2 | 3.5* | 3.5* |
| Saliva only (NC1) | 0.5 | 0 | 0 | 0 | 0 |
| Saliva + Triton X100 (PC1) | 3.5* | 2.5* | 2.5* | 2.5* | 2.5* |

| | | | | | |
|---|---|---|---|---|---|
| Virus titre~10³TCID₅₀/ml * No virus recovery. However, using the Karber Calculation method (taking into account the initial 1/10 dilution) a titre of 0.5 TCID₅₀/ml is obtained. | | | | | |

**Table 2**

| The following table shows the log reduction in viral titre for both the test composition and control compositions in the virucidal assay conducted at 22.9 °C. | | | | | |
|---|---|---|---|---|---|
| | Contact Time | | | | |
| Test/Control | Time (minutes) | | | | |
| Compounds | 0.5 | 1 | 2 | 4 | 8 |
| Strepsils Original Throat Lozenges (TC) | 0.50 | 0.50 | 0.50 | 1.50 | 1.00 |
| Saliva only (NC1) | 0.00 | 0.00 | 0.00 | 0.00 | 0.50 |
| Saliva + Triton X100 (PC1) | 3.0* | 3.92* | 3.92* | 3.92* | 3.92* |

| | | | | | |
|---|---|---|---|---|---|
| Virus titre~10³TCID₅₀/ml * No virus recovery. However, using the Karber Calculation method (taking into account the initial 1/10 dilution) a titre of 0.5 TCID₅₀/ml is obtained. | | | | | |

Cycotoxicity tests employing Urbani SARS virus, test composition (TC), PC1 and NC1 established these compositions were not toxic to C1008 cells during the virucidal assays. The results of Table 1 and Table 2 indicate the ability of a mixture of 2,4-dichlorobenzyl alcohol and 0.6 mg amylmetacresol in an acidic sucrose solution to reduce very significantly the infectivity of Urbani SARS-associated coronavirus. Suitably, these results were supported by performing a cytopathic observation (CPE) in duplicate on each of the samples employing electron microscopy as detailed in Tables 3, 4 and 5 below.

**Table 3: CPE observations from virucidal assay with Strepsils Original Throat Lozenges (TC) at 37°C**

| Time minutes) | Dilution*→ | | | | | | | Cells Only |
|---|---|---|---|---|---|---|---|---|
| | 10⁻² | 10⁻³ | 10⁻⁴ | 10⁻⁵ | 10⁻⁶ | 10⁻⁷ | 10⁻⁸ | |
| 1 | + | +/- | - | - | - | - | - | - |
| 2 | + | - | - | - | - | - | - | - |
| 4 | - | - | - | - | - | - | - | - |
| 8 | - | - | - | - | - | - | - | - |
| Virus control | + | + | +/- | +/- | - | - | - | - |

| | | | | | | | | |
|---|---|---|---|---|---|---|---|---|
| * refers to dilution of residual virus | | | | | | | | |

**Table 4: CPE observations from virucidal assay with Saliva only (NC) at 37 °C**

| Time minutes) | Dilution*→ | | | | | | | Cells Only |
|---|---|---|---|---|---|---|---|---|
| | 10⁻² | 10⁻³ | 10⁻⁴ | 10⁻⁵ | 10⁻⁶ | 10⁻⁷ | 10⁻⁸ | |
| 1 | + | + | +/- | - | - | - | - | - |
| 2 | + | + | +/- | - | - | - | - | - |
| 4 | + | + | +/- | - | - | - | - | - |
| 8 | + | +/- | +/- | - | - | - | - | - |
| Virus control | + | + | - | - | - | - | - | - |

| | | | | | | | | |
|---|---|---|---|---|---|---|---|---|
| * refers to dilution of residual virus | | | | | | | | |

**Table 5: CPE observations from virucidal assay with Saliva and Triton X100 (PC) at 37°C**

| Time minutes) | Dilution*→ | | | | | | | Cells Only |
|---|---|---|---|---|---|---|---|---|
| | 10⁻² | 10⁻³ | 10⁻⁴ | 10⁻⁵ | 10⁻⁶ | 10⁻⁷ | 10⁻⁸ | |
| 1 | - | - | - | - | - | - | - | - |
| 2 | - | - | - | - | - | - | - | - |
| 4 | - | - | - | - | - | - | - | - |
| 8 | - | - | - | - | - | - | - | - |
| Virus control | + | + | - | - | - | - | - | - |

| | | | | | | | | |
|---|---|---|---|---|---|---|---|---|
| * refers to dilution of residual virus | | | | | | | | |

In the above Tables 3 to 5 the symbols have the following meanings:
+ = positive for cytopathic observation in both wells
- = negative for cytopathic observation in both wells
+/- = positive for cytopathic observation in one well and negative for cytopathic observation in the other well

Suitably, the results as detailed in Tables 3 and 4 demonstrate that an acidic sucrose base including 2,4-dichlorobenzyl alcohol and amylmetacresol provides enhanced negative cytopathic observation compared with saliva alone, thereby indicating enhanced virucidal activity against Urbani SARS-associated coronavirus.

### Examples 2 to 9

The following sugar based lozenges were prepared as outlined in Table 4. The ingredients of the final lozenges are expressed in milligrammes.

**Table 4 - Sugar Based Lozenges**

| Ingredient (mg) | Ex. 2 | Ex. 3 | Ex. 4 | Ex. 5 | Ex. 6 | Ex. 7 | Ex. 8 |
|---|---|---|---|---|---|---|---|
| Sucrose | 1400 | 1400 | 1400 | 1300 | 1450 | 1425 | 1450 |
| Glucose | 1100 | 1100 | 1100 | 1150 | 1260 | 1260 | 1200 |
| 2,4-dichlorobenzyl alcohol | 0.5 | 1.2 | 2.5 | 1.5 | 1.5 | 1.6 | 2.8 |
| Amylmetacresol | 0.25 | 0.6 | 1.0 | 0.5 | 0.5 | 0.7 | 0.75 |
| Tartaric acid | - | - | - | - | 4 | - | - |
| Citric acid | - | - | - | - | - | 4.5 | 3.5 |
| Excipient (flavouring, preservative, colouring) | - | - | - | - | - | 3.0 | - |
| Water | Trace amounts | | | | | | |

A mixture of sucrose and liquid glucose (80% by wt glucose and 20% by wt water available from Cerestar, United Kingdom) is heated to 140 °C and a vacuum applied to reduce the water content of the mixture. The excipients are added in a sealed vessel. The 2,4-dichlorobenzyl alcohol and amylmetacresol and acid (where appropriate) are blended and the blend added to the remainder of the ingredients. The resulting mixture is cooled and formed into a continuous cylindrical mass from which the individual lozenges are formed.

### Examples 10 to 17

The following sugar alcohol based lozenges as detailed in Table 5 were prepared in accordance with Example 2, except the sugar base was replaced with the appropriate sugar alcohol and approximately 20% by water added. The resultant mixture is heated to 145 to 170 °C under vacuum to reduce the water content of the mixture, and the other components blended in as described in Example 2.

**Table 5**

| Ingredient (mg) | Ex. 10 | Ex. 11 | Ex. 12 | Ex. 13 | Ex. 14 | Ex. 15 | Ex. 16 | Ex. 17 |
|---|---|---|---|---|---|---|---|---|
| Sorbitol | 2200 | 2300 | - | - | - | - | 2444 | - |
| Isomalt | - | - | 2450 | 1225 | 2455 | 2450 | - | - |
| Lycasin | - | - | - | 1225 | - | - | - | 2301 |
| 2,4-dichlorobenzyl alcohol | 1.6 | 2.9 | 1.6 | 1.6 | 0.9 | 1.2 | 2.9 | 1.8 |
| Amylmetacresol | 0.6 | 2.4 | 0.6 | 0.6 | 0.7 | 0.5 | 0.7 | 0.8 |
| Tartaric acid | - | 4.0 | - | - | 20 | 4.0 | 5.0 | 4.6 |
| Citric acid | - | - | - | - | - | - | - | - |
| Excipient (flavouring, preservative, colouring) | - | 3.0 | - | - | - | 4.0 | 4.2 | 3.8 |
| Water | Trace amounts | | | | | | | |

### Examples 18 to 23

The following suckable or masticable tablets were formed by blending the ingredients as detailed in Table 6 for approximately 15 minutes and compressing the resultant mixture in a tabletting machine.

**Table 6**

| Ingredient (mg) | Ex. 18 | Ex. 19 | Ex. 20 | Ex. 21 | Ex. 22 | Ex. 23 |
|---|---|---|---|---|---|---|
| Sucrose | 2350 | 2340 | 2560 | - | - | - |
| Glucose | - | - | - | 2500 | - | - |
| 2,4-dichlorobenzyl alcohol | 1.5 | 1.8 | 1.7 | 1.2 | 1.2 | 1.2 |
| Amylmetacresol | 0.5 | 0.4 | 0.5 | 0.5 | 0.5 | 0.6 |
| Tartaric acid | - | - | 4.0 | 3.5 | 3.5 | - |
| Excipient (binder, flavouring, preservative, colouring) | - | 4.0 | 4.0 | 3.5 | 4.0 | 3.0 |
| Isomalt | - | - | - | - | 2489 | 2401 |

### Examples 24 to 27

The following solutions, syrups and suspensions were prepared by mixing the respective ingredients at room temperature for 15 minutes.

| Ingredient | Solution | Syrup | Syrup | Suspension |
|---|---|---|---|---|
| | Ex. 24 | Ex. 25 | Ex. 26 | Ex. 27 |
| Amylmetacresol | 0.6 mg | 0.6 mg | 0.6 mg | 0.6 mg |
| 2,4-dichlorobenzyl alcohol | 1.2 mg | 1.2 mg | 1.2 mg | 1.2 mg |
| Excipient (flavouring, preservative, colouring) | 1 mg | 1 mg | 1 mg | 1 mg |
| Water | 4.5 ml | - | - | 4.5 ml |
| Ethanol | 0.5 ml | 0.5 ml | 0.5 ml | - |
| Liquid sucrose (70% by wt sucrose, 30% by wt water) | - | 4.5 ml | - | - |
| Liquid glucose (80% by wt glucose, 20% by wt water) | - | - | 4.5 ml | - |
| Polymeric suspending agent (e.g. guar gum or starch) | - | - | - | 5 mg |

## Claims

1. The use of 2,4-dichlorobenzyl alcohol with amylmetacresol in the manufacture of a medicament for the treatment or prevention of viral infections caused by SARS-associated coronavirus.

2. Use as claimed in claim 1 wherein the SARS-associated coronavirus comprises Urbani SARS-associated coronavirus.

3. Use as claimed in claim 1 or 2 wherein the medicament is adapted for nasal, ocular or oral administration, especially oral administration.

4. Use as claimed in any one of the preceding claims wherein the medicament is in the form of a suckable or masticable solid dosage form.

5. Use as claimed in claim 4 wherein the medicament in the form of a lozenge.

6. Use as claimed in any one of claims 1 to 3 wherein the medicament is in the form of a liquid.

7. Use as claimed in any one of the preceding claims wherein the ratio by weight of amylmetacresol to 2,4-dichlorobenzyl alcohol is in the range of 1:5 to 1:1.

8. Use as claimed in any one of the preceding claims wherein the medicament is acidulated.

9. Use as claimed in any one of the preceding claims wherein the medicament includes an acidulated sugar base, especially an acidulated sucrose and/or glucose base.

10. Use as claimed in any one of the preceding claims wherein the medicament includes an acidulated sugar alcohol base.

## Patentansprüche

1. Verwendung von 2,4-Dichlorbenzylalkohol mit Amylmetacresol bei der Herstellung eines Medikaments zur Behandlung oder Vorbeugung von Virusinfektionen, die durch mit SARS in Zusammenhang stehendem Coronavirus verursacht wird.

2. Verwendung nach Anspruch 1, wobei der mit SARS in Zusammenhang stehende Coronavirus mit Urbani-SARS in Zusammenhang stehenden Coronavirus umfasst.

3. Verwendung nach Anspruch 1 oder 2, wobei das Medikament zur nasalen, okularen oder oralen Verabreichung, insbesondere zur oralen Verabreichung geeignet ist.

4. Verwendung nach einem der vorangehenden Ansprüche, wobei das Medikament in Form einer lutschbaren oder kaubaren festen Dosierungsform vorliegt.

5. Verwendung nach Anspruch 4, wobei das Medikament in Form einer Pastille vorliegt.

6. Verwendung nach einem der Ansprüche 1 bis 3, wobei das Medikament in Form einer Flüssigkeit vorliegt.

7. Verwendung nach einem der vorangehenden Ansprüche, wobei das Gewichtsverhältnis von Amylmetacresol zu 2,4-Dichlorbenzylalkohol im Bereich von 1:5 bis 1:1 liegt.

8. Verwendung nach einem der vorangehenden Ansprüche, wobei das Medikament angesäuert ist.

9. Verwendung nach einem der vorangehenden Ansprüche, wobei das Medikament eine angesäuerte Zuckerbasis, insbesondere eine angesäuerte Saccharose- und/oder Glucosebasis einschließt.

10. Verwendung nach einem der vorangehenden Ansprüche, wobei das Medikament eine angesäuerte Zuckeralkoholbasis einschließt.

## Revendications

1. Utilisation d'alcool 2,4-dichlorobenzylique avec de l'amylmétacrésol dans la production d'un médicament pour le traitement ou la prévention d'infections virales provoquées par un coronavirus associé à SARS.

2. Utilisation suivant la revendication 1, dans laquelle le coronavirus associé à SARS comprend le coronavirus associé à SARS Urbani.

3. Utilisation suivant la revendication 1 ou 2, dans laquelle le médicament est adapté à l'administration nasale, oculaire ou orale, notamment à l'administration orale.

4. Utilisation suivant l'une quelconque des revendications précédentes, dans laquelle le médicament est sous forme d'une forme posologique solide pouvant être sucée ou mastiquée.

5. Utilisation suivant la revendication 4, dans laquelle le médicament est sous forme d'une pastille.

6. Utilisation suivant l'une quelconque des revendications 1 à 3, dans laquelle le médicament est sous forme d'un liquide.

7. Utilisation suivant l'une quelconque des revendications précédentes, dans laquelle le rapport pondéral de l'amylmétacrésol à l'alcool 2,4-dichlorobenzylique est compris dans l'intervalle de 1:5 à 1:1.

8. Utilisation suivant l'une quelconque des revendications précédentes, dans laquelle le médicament est acidulé.

9. Utilisation suivant l'une quelconque des revendications précédentes, dans laquelle le médicament comprend un excipient consistant en sucre acidulé, notamment un excipient consistant en saccharose et/ou glucose acidulé.

10. Utilisation suivant l'une quelconque des revendications précédentes, dans laquelle le médicament comprend un excipient consistant en sucre-alcool acidulé.
